# EUROPEAN PATENT APPLICATION

(11) **EP 1 258 243 A1**
(43) Date of publication of application: **20.11.2002**
(21) Application number: 01201835.4
(22) Date of filing: 16.05.2001
(51) Int. Cl.: A61K 31/385, A61K 47/42, A61P 39/06

(54) **Lipoic acid for suppressing undesired haematological effects of chemotherapy and/or radiotherapy**

(71) Applicant: N.V. Nutricia, 2700 MA Zoetermeer (NL)
(72) Inventor: Van Den Berg, Jeroen.J.M., NL-3971 HC Driebergen (NL); Osanto, Susanne., NL-2341 JB Oegstgeest (NL); Hageman, Robert.J.J., NL-6705 CT Wageningen (NL)
(74) Representative: Jorritsma, Ruurd

(57) **Abstract**

The present invention is concerned with a method of suppressing the detrimental effects of chemotherapy and/or radiotherapy on a patient's health. More specifically the invention relates to a method comprising the administration of a special pharmaceutical or dietetic preparation containing lipoic acid and/or lipoic acid analogue in an effective amount to suppress the reduction in blood cell count resulting from chemotherapy and/or radiotherapy. The invention also relates to a pharmaceutical or dietetic preparation comprising: lipoic acid and/or lipoic acid analogue in an amount which is equivalent to 40-2000 mg R(+) lipoic acid; 0.2-60 µmoles intact protein; 200-800 mg vitamin C; 100-500 mg vitamin D; 200-1000 mgN-acetyl cystein and 5-100 mg zinc.

## Description

### TECHNICAL FIELD

The present invention is concerned with a method of suppressing the detrimental effects of chemotherapy and/or radiotherapy on a patient's health. More specifically the invention relates to a method comprising the administration of a special pharmaceutical or dietetic preparation containing lipoic acid and/or lipoic acid analogue in an effective amount to suppress the reduction in blood cell count resulting from chemotherapy and/or radiotherapy.

### BACKGROUND

In the US alone yearly about 800,000 new cases of cancer are discovered. In most industrialised countries cancer is usually treated by surgically removing the tumour and/or chemotherapy and/or irradiation (radiotherapy). The chemotherapy methods employed make use of agents that display antineoplastic, cytostatic, antibacterial and antiviral properties. Examples of chemotherapy agents that have found wide application are: alkylating agents such as cisplatin, tetrazines or nitrosourea substances, aziridines, musterd substances as well as antibiotics such doxorubicine, mitomycine and bleomycine. In addition anti-metabolites such as folic acid or nucleic acid analogues are used.

It is well known that both chemotherapy and radiotherapy usually lead to a range of undesirable side-effects. Most of these side-effects are the consequence of the cell damage brought about by the chemotherapy agents or irradiation. These side-effects constitute serious drawbacks of chemotherapy and radiotherapy and quite severely limit clinical utility of chemotherapy agents.

Chemotherapy agents usually exhibit very low therapeutic ratios (the ratio of toxic dose to effective-dose), meaning that the extent to which a patient can be treated with such agents is limited by the toxicity level that is deemed acceptable. As a result of the side-effects of chemotherapy and radiotherapy, doses of chemotherapy agent and/or irradiation often have to be reduced. Consequently the cancer treatment is prolonged, or worse, the chance of successful treatment is substantially reduced.

It has become commonplace to monitor the effects of chemotherapy and radiotherapy by analysing haematological parameters, especially blood cell counts, of a patient at regular intervals. Chemotherapy and radiotherapy are normally found to cause very significant reductions in blood cell count. Naturally such a reduction in blood cell count is associated with a worsening of the general health condition of the patient. Indeed such blood cell counts are used as a crucial parameter in chemotherapy to determine which dosage therapy should be followed for a particular patient. If the cell counts drop below a certain critical level, chemotherapy/radiotherapy will be discontinued or dosage levels of the chemotherapy agent or irradiation will be adjusted downwards so as to prevent cell counts from dropping even lower. It is generally accepted that blood cell counts are a reliable indicator of the health damage resulting from chemotherapy and/or radiotherapy.

Much research has been and is being conducted to find new therapies that have a high specificity against tumour cells. In addition a lot of work is done to find ways to reduce the side-effects of chemotherapy and irradiation. Unfortunately, however, so far with limited success. Cocktails of various agents are being tested and haematological parameters such as the number of leukocytes or thrombocytes are evaluated in order to estimate the magnitude of the side-effects. Despite all this work chemotherapy and radiotherapy are still associated with serious side-effects that limit the extent of their application. Because chemotherapy and radiotherapy remain a crucial elements of cancer treatment in general, there is a strongly felt need for agents that would temper the negative health effects resulting from these treatments. Not only would such agents help to alleviate the detrimental effects of these treatments, they would also enable the application of higher doses of chemotherapy agents and/or irradiation, thereby enhancing the chance of a positive result.

Lipoic acid (1,2 dithiolane-3-pentanoic acid or thioctic acid) is know as a pharmaceutical agent that may be used to reduce liver damage, to diminish the negative effects of a high doses of paracetamol or heavy metals. Lipoic acid is also applied in a number of health preparations that mainly consist of herbal extracts and vitamines. In such preparations lipoic acid is incorporated because of its anti-oxidative properties, i.e. its ability to capture radicals *in vitro.* Lipoic acid is also used in the treatment of diabetes, or to be more precise, in the treatment of insulin resistance and neuropathies. It is further known that lipoic acid plays an important role in oxidative decarboxylation processes in mammals.

WO 00/48594 describes a method for destroying tumour cells which method uses lipoic acid in combination with vitamin C. Suppression of reduction in blood cell count is nowhere mentioned in this patent application.

WO 99/06040 describes a method of reducing the incidence of cardiovascular disease and specific types of cancer in tobacco smokers using a combination of a tocotrienol and alpha-lipoc acid or tocotrienyl lipoate.

WO 99/55326 is concerned with a method of increasing glutathion levels in mammals by co-administering vitamin C and N-acetylcysteine. and a range of other components. Preparations for oral administration comprising alpha lipoic acid are described in the examples.

WO 98/57627 describes the combined use of carnitine and lipoic acid in a method of increasing the metabolic rate of aged cells without a concomitant increase in metabolic production of reactive oxygen species.

Rybak et al, reports that impaired hearing in rats as a result cancer therapy can be attenuated by intra-peritoneally administering high quantities of alfa-lipoic acid, a thio-substance or selenium.

### SUMMARY OF THE INVENTION

Applicants have discovered that lipoic acid and its analogues can successfully be employed in a method of suppressing the reduction in blood cell count resulting from chemotherapy and/or radiotherapy, more particularly such a reduction in blood cell count resulting from chemotherapy and/or radiotherapy as applied in cancer treatment. The method according to the present invention offers the advantage that it diminishes the negative (side) effects of chemotherapy and/or radiotherapy which negative effects more often than not have a dramatic effect on the patient's health. The method additionally offers the advantage that it may enable continuation of chemotherapy/radiotherapy in cases where, in the absence of the present method, the therapy would have to be discontinued or dosages would have to be lowered because blood cell counts would otherwise be reduced to unacceptably low levels.

### DETAILED DESCRIPTION OF THE INVENTION

One aspect of the present invention is concerned with the use of lipoic acid and/or lipoic acid analogue in the manufacture of a pharmaceutical or dietetic preparation for use in a method of suppressing the reduction in blood cell count resulting from chemotherapy and/or radiotherapy, said method comprising the administration of lipoic acid and/or lipoic acid analogue in an amount effective to achieve a significant suppression of the reduction in cell count. Here the term chemotherapy encompasses a method of treating patients by administering a antineoplastic, cytostatic, antibacterial and/or antiviral agent.

Lipoic acid exists in 2 isomer forms, i.e. R(+) lipoic acid and L(-) lipoic acid. Both isomers as well as racemic mixtures thereof can suitably be used in the method of the present invention. Lipoic acid is easily reduced to its disulfide form. Both the reduced and oxidised fom of lipoic acid can suitably he used in the method of the invention and are encompassed by the term "lipoic acid". Lipoic acid is quickly absorbed into the blood steam and equally fast removed therefrom. As a result bio-availability after oral administration is usually low. Particularly in fastened subjects it was found that less than 35% of the lipoic acid actually ingested became biologically available.

Here by lipoic acid analogues are to be understood substances that either liberate lipoic acid in the body of the subject to whom the preparation is being administered. Usually this means that as a result of metabolic activity the lipoic acid is converted into lipoic acid or into a lipoic acid metabolite. Likewise the term lipoic acid analogue also encompasses substances that are structurally closely related and that show the same physiological functionality.

The present method may suitably be applied to vertebrate animals. The method most suitable for application in mammals. Most preferably the present method is used to suppress the negative effect of chemotherapy and/or radiotherapy on blood cell count in humans. The method is advantageously used to suppress reduction in blood cell count resulting from cancer chemotherapy.

The amount of lipoic acid or lipoid acid analogue needed to achieve the desired suppression depends on a number of factors such as frequency of administration, the body weight of the subject, the type and dosage of chemotherapy agent employed, the duration of the chemotherapy, the physical condition of the subject etc. In general good results are obtained if the present method comprises administration of lipoic acid and/or lipoic acid analogue in a daily amount which is equivalent to 40-2000 mg R(+) lipoic acid.

The term "blood cell count" as used throughout this document, relates to the numbers of blood cells as determined by analytical methods well known in the art. Examples of blood cells which can suitably be measured by these techniques are: leukocytes, thrombocytes, lymphocytes and neutrophils (granulocytes). The present method aims to reduce the reduction in cell count of at least one of the aforementioned types of blood cells. Preferably the method suppresses the reduction of leukocyte count and/or thrombocyte count and/or granulocyte count. Most preferably the method suppresses the reduction of leukocyte and/or thrombocyte count. Decreases in leukocyte levels are highly undesirable as these increase the risk of neutropene fever, infections, abscess etc. Decreases in thrombocyte count are associated with, for instance, an enhanced risk of blood loss.

The preparations used in the present process may suitably be administered before, during and/or after administration of the chemotherapy agent or before, during and/or after irradiation. Preferably the preparations of the invention are administered after the chemotherapy agent has been administered or irradiation has occurred. The preparation is preferably first administered no more than 48 hours, most preferably no more than 12 hours after said event. It is noted that it is also possible to combine chemotherapy agent and the present preparation into a single dosage units. Such an embodiment is encompassed by the present invention.

The method according to the present invention is effective in suppressing the reduction in blood cell count caused by a wide variety of chemotherapy agents. Such chemotherapy agents include:
- 1.: Alkylating agents (e.g. aziridine, alkyl sulfonates, nitrosourea, tetrazines, cisplatin, carboplant, procarbazine, hexamethylmelamine, adozelesin)
- 2.: Antimetabolites (e.g. folic acid analogues such as methorexate, antifolates such trimetrexate and tomudex, pyrimidine analogues, purine analogues and substituted urea substances such as hydroxy urea)
- 3.: Natural antibiotics (e.g. bleomycin, dactinomycin, daunorubicin, doxorubicin, mithramycin, mitomycin)
- 4.: Natural mitotic inhibitors (e.g. vincristine, vinblastine, vindesine, vinorelabine)
- 5.: Podophyllum derivatives (e.g. etoposide, teniposide)
- 6.: Cemptothecin analogues such as topotecan
- 7.: Taxanes (paclitaxel and docetaxel)
- 8.: Enzymes such as asparaginase
- 9.: Hormone and hormone antagonists (e.g. androgen, corticosteroids, estrogens, progestins, estrogen antagonists)
- 10.: Topoisomerase interactive agents (e.g. idarubicin, epirubicin, mitoxantrone, losoxantrone, amsacrine, pyrazoloacridine)
- 11.: Retinoids (e.g. all trans-retinoic acids, 13-cis retinoic acid, 9-cis retinoic acid, selective retinoid receptor agonists, fenretinide, 14-hydroxy-retro-retinol)
- 12.: Methyl hydrazine derivatives such as porcarbazine
- 13.: Adrenal cortical suppressants such as mitotane
- 14.: Steroid synthesis inhibitor such as aminoglutethimide

The present method is particularly effective in suppressing undesirable health effects resulting from a chemotherapy agent selected from the group consisting of alkylating agents and antimetabolites.

The pharmaceutical or dietetic preparation used in the present method may be administered in different ways, well-known in the art. It was found to be beneficial to administer the preparation orally. Oral preparations for use in the present method advantageously contain a lipophylic carrier. Examples of suitable lipophylic carriers are glycerides, phospholipids, cholesterol, glycerol, polyethylene glycol and mixtures thereof. Here the term glycerides encompasses triglycerides (e.g. MCT oil), diglycerides and monoglycerides. Most preferably the lipophylic carrier is solid at ambient. A suitable example of such a carrier is triglyceride fat containing a relatively high saturated fatty acid moiety. It is noted that consumption of large amounts of lipophylic material such as fat, may interfere with the resorption of lipoic acid and/or lipoic acid analogue. Hence the daily amount of the present preparation preferably contains less than 10 g of lipophylic carrier.

In an even more preferred embodiment the oral preparation used in the present method is a sustained release preparation which releases most of the lipoic acid and lipoic acid analogue in or after the duodenum. Such a sustained release preparation may suitably consist of microencapsules. Also coated tablets, in particular tablets coated with an acid polymer, can advantageously be employed as sustained release preparation.

In a preferred embodiment of the invention the lipoic acid analogue used is selected from the group consisting of pharmaceutically acceptable salts of lipoic acid, esters of lipoic acid, amino acid-adducts of lipoic acid, peptide adducts of lipoic acid, lipoamide and mixtures thereof. The esters of lipoic acid preferably comprise an acyl group with no more than 8 carbon atoms, preferably with less than 4 carbon atoms. Examples of adducts of lipoic acid that can suitably be applied as lipoic acid analogues in accordance with the invention are adducts of lipoic acid and lysine and adducts of lipoic acid and lysine-rich peptides.

In a particularly preferred embodiment of the invention the method comprises the administration of the oxidised form of lipoic acid in an amount effective suppress the undesirable side effects. It was found that the oral administration of preparations containing oxidised lipoic acid is easier for patients suffering from mucositis. Mucositis is a side effect often encountered in patients undergoing chemotherapy. The daily administered amount of oxidised lipoic acid is preferably kept below 10 mg/kg of bodyweight.

It was found that the low bio-availability of lipoic acid (bioavailability of R- and L-isomer are about 38% and 28% respectively), may be improved by co-administration of a suitable amount of intact protein. Consequently, in a preferred embodiment of the invention, the method comprises additional administration of intact protein. The combined use of lipoic acid and intact protein offers the additional advantage that the bad taste of lipoic acid is effectively masked. Also the lipoic acid will cause less irritation to mucosa when co-administered with an adequate amount of intact protein. Preferably the intact protein is administered in an amount effective to increase bio-availability of the lipoic acid or lipoic acid analogue by at least 10%. In general a significant improvement in terms of bio-availability is observed when the present method comprises administration of intact protein in a daily amount of at least 0.1 µmoles, more preferably from 0.2 - 60 µmoles. The term "intact protein" encompasses polypeptides that comprise a relatively high fraction of basic amino acids such as lysine and arginine. Preferably the lysine content of the intact protein is at least 8%. The cystein content of the protein is preferably at least 2%.

In order to obtain best results from the combined administration of lipoic acid and intact protein, it was found beneficial to apply these 2 components in specific ratios. Hence in a preferred embodiment of the invention the method comprises administration of lipoic acid and/or lipoic acid analogue and intact protein in a ratio of between 10:1 and 2,000:1 lipoic acid equivalent/µmole intact protein.

Particularly good results are obtained if the present method uses intact protein in the form of transferrin. Preferably the transferrin used in the present method is selected from the group selected from the group consisting of lactoferrin, conalbumin, (lacto)ferricin and mixtures thereof

In another preferred embodiment the intact protein comprises metal and/or alkalimetal ions in an amount of at least 20%, more preferably at least 50% of the maximum number of metal and/or alkalimetal ions that the protein may hold (the saturation level). The aforementioned metal ions are preferably iron cations.

In accordance with the present invention lactoferrin and conalbumin are suitably adminstered in daily amounts of 14-5000 mg, preferably 100-1000 mg. Likewise (lacto)ferricin is suitably administered in daily amounts of 2-500 mg, preferably 10-100mg.

The combined use of lipoic acid and intact protein has the advantage that less lipoic acid needs to be administered to achieve the same level of suppression that would be observed if lipoic acid were administered in the absence of intact protein. This is relevant as lipoic acid is know to have *in vitro* cytotoxic effects at concentrations above 0.2 mM. Given that many cancer patients suffer from mucositis of the gatrointestinal tract it is deemed advisable to apply even lower dosage levels of lipoic acid or lipoic acid analogue in such patients. The combined application of lipoic acid and intact protein makes it possible to reduce the daily dosage level of lipoic acid to less than 1000 mg, whilst still achieving significant suppression of reduction in blood cell count. Hence in a very preferred embodiment of the invention the method comprises administration of lipoic acid and/or lipoic acid analogue in a daily amount which is equivalent to 40-1000 mg R(+) lipoic acid. More preferably said dosage levels are within the range of 50-600 mg R(+) lipoic acid.

The lipoic acid used in the method of the invention may be obtained by organic synthesis, through natural production processes such as fermentation and enzymolysis, or by means of isolation and/or extraction from natural plant and animal materials. Examples of such natural raw materials are potato, spinach, eggs, yeast, heart and liver tissue.

The preparations used in the present method may also advantageously contain vitamin C, vitamin E, thiamine (e.g. as thiamine.HCl), flavonoids, carotenoids, zinc, manganese, magnesium, copper, selenium and/or coenzyme Q10. It is preferred to include thiamine in an amount of 0.5-5 mg per daily dose as this will further improve the overall efficacy of the present method. Preferably, if present, the following components are co-administered in daily amounts which are within the ranges cited below:

| | Minimum daily dosage | Maximum daily dosage |
|---|---|---|
| Vitamin C | 200 mg | 800 mg |
| Vitamin E | 100 mg | 500 mg |
| Vitamin B1 | 0.5 mg | 5 mg |
| N-acetyl cystein | 200 mg | 1000 mg |
| Zinc | 5 mg | 100 mg |
| Manganese | 2 mg | 8 mg |
| Copper | 1 mg | 10 mg |
| Selenium | 0.05 mg | 0.2 mg |
| Coenzyme Q10 | 20 mg | 140 mg |

The daily amount of magnesium administered in accordance with the present preparation preferably lies within the range of 50-400 mg, more preferably between 100 and 300 mg.

In a particularly preferred embodiment of the invention, the preparation comprises vitamin E, vitamin C, N-acetyl cystein and zinc in the above mentioned amounts. Even more preferably the preparation also contains vitamin B1 in the above mentioned amount.

It should be understood that the invention also encompasses the use of functional analogues of the above components. In the case of such analogues the minimum and maximum daily dosage can be established by determining which concentrations produce a functionally equivalent result to the minimum and maximum daily dosage cited for the parent component in the above table.

The dietetic preparations for use in the present method may suitably take the form of a beverage, a liquid (dilutable) concentrate, a water-soluble powder or granulate, or a candy bar. The pharmaceutical preparation may take the form of a tablet, a powder, a capsule, a solution, an emulsion, a suspension or a suppository.

Another aspect of the invention relates to a pharmaceutical or dietetic preparation comprising:
- a.: lipoic acid and/or lipoic acid analogue in an amount which is equivalent to 40-2000 mg R(+) lipoic acid,
- b.: 0.2-60 µmoles intact protein,
- c.: 200-800 mg vitamin C,
- d.: 100-500 mg vitamin E,
- e.: 200-1000 mg N-acetyl cystein
- f.: 5-100 mg zinc.
The aforementioned preparation may advantageously and additionally comprise between 0.5 and 5 mg vitamin B1.

The invention is further illustrated, but not limited to the embodiments described in the following examples.

### EXAMPLES

### Example 1

A study was conducted using a group of 140 patients suffering from various forms of cancer. All these patients started participating in this study when they were first going to be subjected chemotherapy and/or radiotherapy. Chemotherapy agents used in these treatments were: cisplatin and doxorubicine. The effect of chemotherapy and radiotherapy on blood cells was monitored by measuring cell counts for neutrophilic granulocytes: leukocytes and thrombocytes at regular intervals.

The 140 patients were randomly divided into 2 groups of each 70 patients. To one group only placebos (9 tablets per day) were administered. The other group received 9 tablets per day, which tablets had the following composition:

| Recommended daily dosage levels | | |
|---|---|---|
| Vitamin E | 50 mg | 100-500 mg |
| Vitamin C | 80 mg | 200-800 mg |
| Alpha lipoic acid | 50 mg | |
| Lactoferrine | 20 mg | |
| N-acetyl cystein | 60 mg | 200-1000 mg |
| Coenzyme Q10 | 15 mg | 20-140 mg |
| Zinc | 1.14 mg | 5-100 mg |
| Copper | 0.26 mg | 1-10 mg |
| Manganese | 0.41 mg | 2-8 mg |
| Selenium | 14 µg | 0.05-0.2 mg |
| Calcium | 20 mg | 50-300 mg |
| Magnesium | 17 mg | 50-400 mg |

For each patient administration of the tablets commenced 3 days before the start of the first cycle of chemotherapy and was continued till at least 3 weeks after the last cycle, with a minimum of 6 cycles. This means that all patients participated for at least 10 weeks in the study.

When comparing the blood cell decreases observed in both groups as a result of chemotherapy it was found that the group that had received tablets with the above composition showed on average a 20% lower decrease than the placebo group.

**Example 2**

| A tablet for oral administration was prepared from the following components: | |
|---|---|
| D,L 6,8-thioctic acid | 60 mg |
| Conalbumine (ex egg) | 40 mg |
| Zinc sulphate | 6 mg |
| MCT oil | 100 mg |
| Thiamine.HCl | 0.4 mg |
| Excipient & conventional additives | |

The above tablet can suitably be used in the method of the present invention.

**Example 3**

| A powder was prepared from the following components: | |
|---|---|
| R,S alpha-lipoic acid | 100 mg |
| Bovine whey protein | 2 g (providing about 40 mg lactoferrin) |
| Maltodextrin | 2 g |
| Zinc sulphate | 30 mg |
| Vitamin E in soybean oil | 600 mg (providing 200 mg vitamin E) |
| Thiamine | 4 mg |
| Silicate | 0.5 g |

The powder was packed in sachets which can be dissolved in a liquid or pudding which may be administered/consumed in accordance with the method of the invention.

## Claims

1. Use of lipoic acid and/or lipoic acid analogue in the manufacture of a pharmaceutical or dietetic preparation for use in a method of suppressing the reduction in blood cell count resulting from chemotherapy and/or radiotherapy, said method comprising the administration of lipoic acid and/or lipoic acid analogue in an amount effective to achieve a significant suppression of the reduction in cell count.

2. Use according to claim 1, wherein the method comprises administration lipoic acid and/or lipoic acid analogue in a daily amount which is equivalent to 40-2000 mg R(+) lipoic acid.

3. Use according to claim 1 or 2, wherein the preparation is for use in a method of suppressing the reduction in leukocyte count and/or thrombocyte count.

4. Use according to any one of claims 1-3, wherein the method comprises the administration of oxidised lipoic acid in an amount effective to achieve a significant suppression of the reduction in cell count.

5. Use according to any one of claims 1-4, wherein the pharmaceutical or dietetic preparation is administered orally.

6. Use according to any one of claims 1-5, wherein the preparation is a sustained release preparation which releases most of the lipoic acid and/or lipoic acid analogue in or after the duodenum.

7. Use according to any one of claims 1-6, wherein the method comprises additional administration of intact protein containing at least 8% lysine, preferably an intact protein in the form of transferrin.

8. Use according to claim 7, wherein the method comprises administration of intact protein in a daily amount of at least 0.1 µmoles, more preferably from 0.2 - 60 µmoles.

9. Use according to any one of claims 6-8, wherein the method comprises administration of lipoic acid and/or lipoic acid analogue and intact protein in a ratio of between 10:1 and 2000:1 mg lipoic acid equivalent/µmole intact protein.

10. Use according to any one of claims 6-9, wherein the transferrin is selected from the group selected from the group consisting of lactoferrin, conalbumin, (lacto)ferricin and mixtures thereof.

11. Use according to any one of claims 6-10, wherein the method comprises administration of lipoic acid and/or lipoic acid analogue in a daily amount which is equivalent to 40-1000 mg, preferably 50-600 mg R(+) lipoic acid.

12. Use according to any of claims 1-11, wherein the method comprises additional administration of daily amounts of 200-800 mg vitamin C, 100-500 mg vitamin E, 0.5-5 mg vitamin B1, 5-100 mg zinc, 2-8 mg manganese, 50-400 mg magnesium, 1-10 mg copper, 0.05-0.2 mg selenium and/or 20-140 mg coenzyme Q10.

13. Pharmaceutical or dietetic preparation comprising:
a. lipoic acid and/or lipoic acid analogue in an amount which is equivalent to 40-2000 mg R(+) lipoic acid,
b. 0.2-60 µmoles intact protein,
c. 200-800 mg vitamin C,
d. 100-500 mg vitamin D,
e. 200-1000 mgN-acetyl cystein
f. 5-100 mg zinc.

14. Preparation according to claim 13, wherein the preparation additionally comprises between 0.5 and 5 mg vitamin B1.
